(19) Europäisches Patentamt
European Patent Office
Office européen des brevets

(11) Veröffentlichungsnummer: **0 208 932**
**A2**

# (12) EUROPÄISCHE PATENTANMELDUNG

(21) Anmeldenummer: 86108217.0

(22) Anmeldetag: 16.06.86

(51) Int. Cl.⁴: C 07 D 277/22, C 07 C 103/54, C 07 C 125/065, C 07 D 295/08, C 07 C 125/073, C 07 D 233/60, A 61 K 31/425, A 61 K 31/14, A 61 K 31/16, A 61 K 31/445, A 61 K 31/415

(30) Priorität: 18.06.85 CH 2567/85

(43) Veröffentlichungstag der Anmeldung: 21.01.87 Patentblatt 87/4

(84) Benannte Vertragsstaaten: AT BE CH DE FR GB IT LI LU NL SE

(71) Anmelder: F. HOFFMANN-LA ROCHE & CO. Aktiengesellschaft, CH-4002 Basel (CH)

(72) Erfinder: Barner, Richard, Dr., Traubenweg 16, CH-4108 Witterswil (CH)
Erfinder: Burri, Kasper, Dr., Im Lichs 3, CH-4310 Rheinfelden (CH)
Erfinder: Cassal, Jean-Marie, Dr., 4 rue du Markstein, F-68100 Mulhouse (FR)
Erfinder: Hadvary, Paul, Dr., Neumattenweg 8, CH-4105 Biel-Benken (CH)
Erfinder: Hirth, Georges, 7 rue de l'ancre, F-68330 Huningue (FR)
Erfinder: Müller, Klaus, Dr., Grellingerstrasse 5, CH-4142 Münchenstein (CH)

(74) Vertreter: Lederer, Franz, Dr. et al, Vanderwerth, Lederer & Riederer Patentanwälte Lucile-Grahn-Strasse 22, D-8000 München 80 (DE)

## (54) Glycerinderivate.

(57) Es werden neue Glycerinderivate der Formel

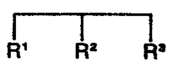

I

worin

einer der Reste $R^1$, $R^2$ und $R^3$ eine Gruppe U der Formel

$OY^1$ oder $-X^1-C(O)-(A^1)_n-Z^1$,

ein weiterer eine Gruppe V der Formel

$OY^2$ oder $-X^2-C(O)-(A^2)_p-Z^2$

und der verbleibende eine Gruppe W der Formel

$-X^3T-(C_{2-6}\text{-Alkylen})-N^+R \quad A^-$ ist,

wobei eines von $X^1$, $X^2$ und $X^3$ Sauerstoff oder $NQ^1$ ist und die zwei anderen Sauerstoff sind,

und die restlichen Symbole die in der Beschreibung angegebene Bedeutung haben,

und deren Hydrate, durch Einführung oder Ergänzung der Reste $R^1$, $R^2$ und $R^3$ in entsprechenden Glycerinderivaten hergestellt.

Die erhaltenen Verbindungen haben den Blutplättchen-aktivierungsfaktor bzw. das Wachstum von Tumoren hemmende Wirkung.

EP 0 208 932 A2

16. Juni 1986

F.HOFFMANN-LA ROCHE & CO. Aktiengesellschaft, Basel/Schweiz

RAN 4045/4

# Glycerinderivate

Die vorliegende Erfindung betrifft neue Glycerinderivate, Verfahren zu ihrer Herstellung und Arzneimittel auf der Basis jener Glycerinderivate.

Die erfindungsgemässen Glycerinderivate sind Verbindungen der Formel

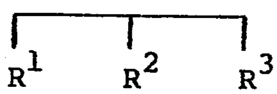

I

worin einer der Reste $R^1$, $R^2$ und $R^3$ eine Gruppe U der Formel $OY^1$ oder $-X^1-CO-(A^1)_n-Z^1$,
ein weiterer eine Gruppe V der Formel
$OY^2$ oder $-X^2-CO-(A^2)_p-Z^2$,
und der verbleibende eine Gruppe W der Formel
$-X^3T-(C_{2-6}\text{-Alkylen})-N^+R$ $A^-$ ist,
wobei eines von $X^1$, $X^2$ und $X^3$ Sauerstoff oder eine Gruppe $NQ^1$ ist und die zwei anderen Sauerstoff sind,

$Y^1$ $C_{10-26}$-Alkyl oder $C_{10-26}$-Alkenyl,

$Y^2$ $C_{1-6}$-Alkyl, $C_{2-6}$-Alkenyl, $C_{3-6}$-Cycloalkyl, $C_{5-6}$-Cycloalkenyl, Phenyl, Benzyl oder 2-Tetrahydropyranyl,

Mé/1.4.86

$A^1$ und $A^2$ Sauerstoff oder eine Gruppe $NQ^2$,
n und p die Zahl 1 oder O,
$Z^1$ $C_{9-25}$-Alkyl oder $C_{9-25}$-Alkenyl,
$Z^2$ $C_{1-5}$-Alkyl, $C_{2-5}$-Alkenyl, Phenyl oder, falls $A^2$ nicht Sauerstoff ist, auch Wasserstoff bedeutet,
T Carbonyl, COO oder $CONQ^3$ oder, falls $X^3$ Sauerstoff ist, auch Methylen bedeutet,
$Q^1$, $Q^2$ und $Q^3$ Wasserstoff, $C_{1-4}$-Alkyl, $C_{3-6}$-Cycloalkyl oder Phenyl,
$A^-$ das Anion einer starken Säure,
$-N^+R$ eine Gruppe $-N^+(Y^3,Y^4,Y^5)$ oder, falls zumindest eines von $X^1$, $X^2$, $X^3$, $A^1$, $A^2$ und T ein substituiertes N-Atom enthält, auch eine am quartären Stickstoff gebundene 5- oder 6-gliedrige aromatische Gruppe, gegebenenfalls mit einem zusätzlichen Heteroatom O, S oder N, gegebenenfalls mit ankondensiertem Benzol und gegebenenfalls monosubstituiert durch Alkyl oder Alkoxy mit bis zu 4 C-Atomen, Hydroxy, Nitro, Carbamoyl oder Ureido,
$Y^3$ und $Y^4$ $C_{1-6}$-Alkyl oder zusammen $C_{3-6}$-Alkylen,
und $Y^5$ $C_{1-6}$-Alkyl sind,
wobei, falls gleichzeitig $R^1$ eine Gruppe $OY^1$ oder $OY^2$, und $R^3$ eine Gruppe W ist, worin $X^3$ Sauerstoff und T Methylen oder Carbonyl bedeutet, $R^2$ die Gruppe OCOO oder ein substituiertes N-Atom enthält,
und deren Hydrate.

Die hier verwendeten Ausdrücke "Alkyl" und "Alkenyl" beziehen sich auf geradkettige oder verzweigte, gesättigte bzw. monoungesättigte Reste, wie Methyl, Aethyl, Propyl, Isopropyl, 2-Propenyl, Butyl, Isobutyl, Hexadecyl, Heptadecyl, Octadecyl und Octadecenyl, insbesondere Methyl und Octadecyl. Beispiele von $C_{3-6}$-Cycloalkylresten sind Cyclopropyl und Cyclohexyl, von $C_{5-6}$-Cycloalkenylresten 2-Cyclopentenyl und insbesondere 2-Cyclohexenyl.

$C_{2-6}$-Alkylengruppen können geradkettig oder verzweigt sein. Beispiele davon sind n-Butylen, 2-Methylpropylen und insbesondere Aethylen und Propylen. Beispiele von Resten $-N^{+}(Y^3, Y^4, Y^5)$ sind 1-Methylpiperidinium und Trimethylammonium. Beispiele von heterocyclischen Gruppen $N^{+}R$ sind Oxazolium, Isoxazolium, Pyridinium, Pyridazinium, Chinolinium, Isochinolinium, und insbesondere N-Methylimidazolium und Thiazolium.

Beispiele für Anionen starker organischer oder anorganischer Säuren sind $C_{1-4}$-Alkylsulfonyloxy, Phenylsulfonyloxy, Tosyloxy, Campher-10-sulfonyloxy bzw. $Cl^-$, $Br^-$, $J^-$, $ClO_4^-$, $SO_4^{--}$, $PO_4^{---}$ und $NO_3^-$.

Die Verbindungen der Formel I können hydratisiert sein. Die Hydratisierung kann im Zuge des Herstellungsverfahrens erfolgen oder allmählich als Folge hygroskopischer Eigenschaften einer zunächst wasserfreien Verbindung der Formel I auftreten.

Die Verbindungen der Formel I enthalten zumindest ein asymmetrisches C-Atom und können somit als optisch aktive Enantiomere, als Diastereomere oder als Gemische davon, z.B. als Racemate, vorliegen.

Bevorzugte Verbindungen der Formel I sind diejenigen, worin $R^1$ eine Gruppe U, $R^2$ eine Gruppe V und $R^3$ eine Gruppe W ist.

Besonders bevorzugte Verbindungen der Formel I sind diejenigen, worin $R^1$ Octadecanoyloxy, Octadecylcarbamoyloxy, Octadecyloxy, Octadecyloxyformamido, N-Isopropyloctadecyloxyformamido oder 1-Isopropyl-3-octadecylureido, und/oder $R^2$ Acetamido, Methoxyformamido, Methoxy, Methylcarbamoyloxy oder Methoxycarbonyloxy und/oder $R^3$ [4-(Trimethylammonio)-n-butyryloxy]chlorid, [2-(1-Methylpiperidinio)äthoxycarbonyloxy]chlorid, [4-(3-Thiazolio)-n-butoxy]bromid, [4-(Trimethylammonio)-n-butoxy]bromid oder [4-(3-Methylimidazolio)-n-butoxy]bromid ist.

Die Verbindungen der Formel I und ihre Hydrate können dadurch hergestellt werden, dass man

a) eine Verbindung der Formel

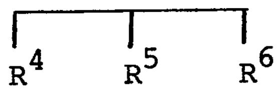 II

worin die Reste $R^4$, $R^5$ und $R^6$ die gleiche Bedeutung wie die Reste $R^1$, $R^2$ bzw. $R^3$ haben, wobei jedoch in der Gruppe W an Stelle des Restes $-N^+R$ $A^-$ eine Abgangsgruppe vorliegt,

mit einem Amin der Formel NR umsetzt, oder

b) eine Verbindung der Formel

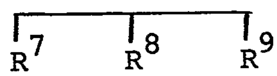 III

worin die Reste $R^7$, $R^8$ und $R^9$ die gleiche Bedeutung wie die Reste $R^1$, $R^2$ bzw. $R^3$ haben, wobei jedoch an Stelle einer der Gruppen U und V eine Hydroxy- oder eine Aminogruppe vorliegt,

mit einer Säure der Formel

$Z^1-(A^1)_n-COOH$ oder $Z^2-(A^2)_p-COOH$ IV

oder einem reaktiven Derivat davon, oder mit einem Isocyanat der Formel

$Z^1NCO$ oder $Z^2NCO$ V

oder einem Imidazolid der Formel

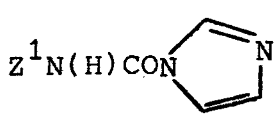 oder 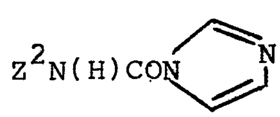 IV

umsetzt, oder

c) eine Verbindung der Formel

$$\overline{R^{O1} \quad R^{O2} \quad R^{O3}} \qquad VII$$

worin $R^{O1}$, $R^{O2}$ und $R^{O3}$ die gleiche Bedeutung wie die Reste $R^1$, $R^2$ bzw. $R^3$ haben, wobei jedoch in der Gruppe W an Stelle der Gruppe $-N^+R$ $A^-$ eine Gruppe $-N(Y^3,Y^4)$ vorliegt,

mit einem $C_{1-6}$-Alkylhalogenid umsetzt, wobei $R^1$, $R^2$, $R^3$, $-N^+R$, $A^-$, U, V, W, $Z^1$, $Z^2$, $A^1$, $A^2$, $Y^3$, $Y^4$, n und p die oben angegebene Bedeutung haben.

Beispiele von in den Verbindungen der Formel II enthaltenen Abgangsgruppen sind Chlor, Brom, Jod, Mesyloxy, Phenylsulfonyloxy und Tosyloxy. Die Reaktion einer Verbindung II mit einem Amin NR kann man in an sich bekannter Weise, z.B. bei einer Temperatur bis zu Rückflusstemperatur des Reaktionsgemisches, zweckmässig bei etwa 80°C, gegebenenfalls in einem Lösungsmittel, wie Acetonitril, Nitromethan oder einem aromatischen Kohlenwasserstoff, z.B. Toluol oder Benzol, oder in Tetrahydrofuran, durchführen.

Beispiele von reaktiven Derivaten der Säuren der Formel IV sind Säurebromide oder Säurechloride und Anhydride. Die Reaktion einer solchen Säure oder eines ihrer reaktiven Derivate mit einer Verbindung der Formel III kann in an sich bekannter Weise durchgeführt werden. Ein Säurechlorid oder Säurebromid kann in einem Lösungsmittel in Gegenwart einer Base bei einer Temperatur von etwa 0 bis 80°C mit der Verbindung III umgesetzt werden. Ein Anhydrid kann in Gegenwart eines Katalysators, wie Dimethylaminopyridin, zweckmässig in einem Lösungsmittel mit der Verbindung III

umgesetzt werden. Als Lösungsmittel können halogenierte Kohlenwasserstoffe, wie Chloroform oder Dichloräthan, und als Basen organische Basen, wie Triäthylamin, Chinolin oder Pyridin, oder anorganische Basen, wie Alkali- oder Erdalkalimetallhydroxyde, -carbonate oder -bicarbonate, z.B. $Na_2CO_3$, $KHCO_3$ oder $Ca_2CO_3$, verwendet werden.

Die Reaktion einer Verbindung der Formel III mit einem Isocyanat der Formel V oder mit einem entsprechenden Imidazolid der Formel VI kann in einem Lösungsmittel, wie Chloroform, Aceton oder Dimethylformamid, bei einer Temperatur zwischen etwa 0 und 100°C, vorzugsweise bei etwa 40-60°C, zweckmässig in Gegenwart eines Katalysators, wie einer Lewis-Base, z.B. Triäthylamin oder Diisopropyläthylamin, durchgeführt werden. Die Reaktion kann gegebenenfalls auch ohne Zusatz eines Lösungsmittels durchgeführt werden.

Die Reaktion eines Amins der Formel VII mit einem $C_{1-6}$-Alkylhalogenid kann man in an sich bekannter Weise, z.B. in einem Lösungsmittel, wie Dichlormethan oder Chloroform, bei einer Temperatur zwischen Raumtemperatur und 50°C, vorzugsweise bei Raumtemperatur, durchführen. Als Halogenid verwendet man vorzugsweise das Jodid.

Die Verbindungen der Formel II können aus denjenigen der Formel IX oder X , und die Verbindungen der Formel III aus denjenigen der Formel VIII nach dem folgenden Reaktionsschema hergestellt werden:

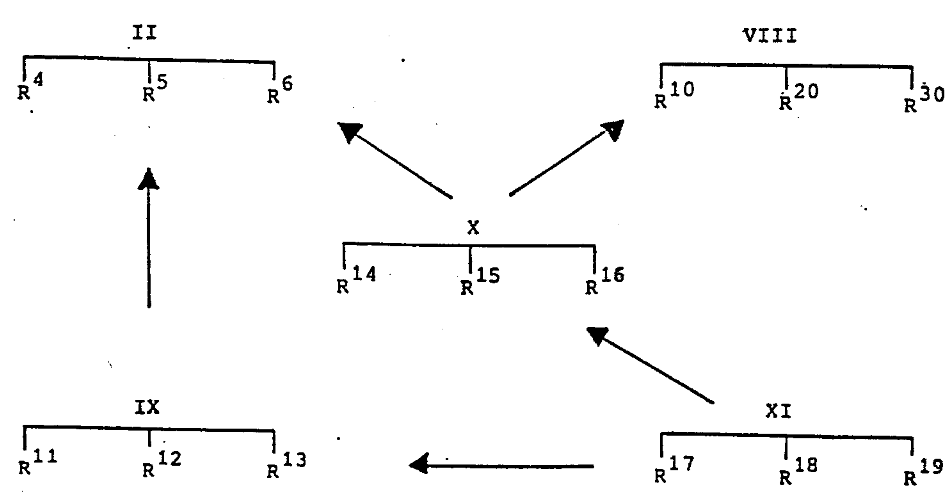

In den Verbindungen der Formel VIII haben die Reste $R^{10}$, $R^{20}$ und $R^{30}$ die gleiche Bedeutung wie die Reste $R^1$, $R^2$ und $R^3$, wobei jedoch an Stelle einer der Gruppen U oder V eine gegebenenfalls geschützte Hydroxy- oder Aminogruppe oder eine Azidogruppe vorliegt.

In den Verbindungen der Formel IX haben die Reste $R^{11}$, $R^{12}$ und $R^{13}$ die gleiche Bedeutung wie die Reste $R^1$, $R^2$ und $R^3$, wobei jedoch an Stelle der Gruppe W eine gegebenenfalls geschützte Hydroxy- oder Aminogruppe oder eine Azidogruppe vorliegt.

In den Verbindungen der Formel X ist einer der Reste $R^{14}$, $R^{15}$ und $R^{16}$ eine gegebenenfalls geschützte Hydroxy- oder Aminogruppe oder eine Azidogruppe, ein weiterer ist eine Gruppe U oder V, und der verbleibende ist eine Gruppe W worin an Stelle von $-N^+R$ $A^-$ eine Abgangsgruppe vorliegt.

In den Verbindungen der Formel XI ist einer der Reste $R^{17}$, $R^{18}$ und $R^{19}$ eine gegebenenfalls geschützte Hydroxy- oder Aminogruppe oder eine Azidogruppe, ein weiterer ist eine gegebenenfalls geschützte Hydroxygruppe, und der verbleibende ist eine Gruppe U oder V.

Beispiele von geschützten Hydroxy- und Aminogruppen sind Aethergruppen, wie Benzyloxy, Trityloxy oder 2-Tetrahydropyranyloxy, bzw. Succinimid, Phthalimid, Benzyloxycarbonylamino oder t-Butoxycarbonylamino.

Zur Herstellung einer Verbindung der Formel II kann man eine Verbindung der Formel IX, worin z.B. $R^{13}$ Hydroxy ist, mit einem Halogenid der Formel Hal-T-($C_{2-6}$-Alkylen)-G, worin G eine Abgangsgruppe, Hal ein Halogenatom ist und T die obige Bedeutung hat, in Gegenwart einer Base, wie Pyridin, oder mit einem Isocyanat der Formel O=C=N-($C_{2-6}$-

-Alkylen)-G umsetzen.

Alternativ kann man eine Verbindung der Formel X, worin z.B. $R^{14}$ Hydroxy ist, in die entsprechende Verbindung der Formel II, worin $R^4$ eine Gruppe U oder V ist, überführen, was in der gleichen Weise wie die weiter oben beschriebene Umwandlung einer Verbindung III in eine Verbindung I bewerkstelligt werden kann. Analog kann man eine Verbindung der Formel XI, worin z.B. $R^{17}$ Hydroxy und $R^{19}$ eine geschützte Hydroxy- oder Aminogruppe ist, in die entsprechende Verbindung der Formel IX, worin $R^{11}$ eine Gruppe U oder V ist, überführen.

Eine in einer Verbindung der Formel IX enthaltene Azidogruppe oder geschützte Hydroxy- oder Aminogruppe, z.B. $R^{13}$, kann in an sich bekannter Weise in die freie Hydroxy- oder Aminogruppe umgewandelt werden. Eine Benzylgruppe kann durch Hydrogenolyse, z.B. über Palladium, abgespalten werden, eine Tritylgruppe mittels Trifluoressigsäure oder verdünnter Salzsäure, und eine 2-Tetrahydropyranylgruppe mittels verdünnter Salzsäure. Eine Azidogruppe kann mit einem komplexen Hydrid, wie Lithiumaluminiumhydrid, oder mittels $H_2$/Pd-C in die Aminogruppe übergeführt werden. Analog kann eine in einer Verbindung der Formel VIII, X oder XI enthaltene Azidogruppe oder geschützte Hydroxy- oder Aminogruppe in die freie Hydroxy- oder Aminogruppe umgewandelt werden. Auf diese Weise wird eine Verbindung der Formel VIII, worin z.B. $R^{10}$ eine geschützte Hydroxy- oder Aminogruppe ist, in die entsprechende Verbindung der Formel III, worin $R^7$ Hydroxy ist, übergeführt.

Zur Herstellung einer Verbindung der Formel X, worin z.B. $R^{16}$ eine Gruppe W ist, in der eine Abgangsgruppe an Stelle von $-NR^+$ $A^-$ vorliegt, kann man die entsprechende Verbindung der Formel XI, worin $R^{19}$ Hydroxy ist, in der gleichen Weise behandeln wie weiter oben für die Ueberführung einer Verbindung IX in eine solche der Formel

II beschrieben.

Die Ueberführung einer Verbindung der Formel X in eine solche der Formel VIII kann in der gleichen Weise bewerkstelligt werden wie die Ueberführung einer Verbindung II in eine Verbindung I.

Man kann eine Verbindung der Formel VII ausgehend von der entsprechenden Verbindung der Formel IX herstellen, worin die Reste $R^{11}$, $R^{12}$ und $R^{13}$ die gleiche Bedeutung wie die Reste $R^1$, $R^2$ und $R^3$ haben, wobei jedoch an Stelle der Gruppe W eine Hydroxy- oder Aminogruppe vorliegt. So kann man eine Verbindung der Formel IX, worin z.B. $R^{13}$ Hydroxy ist, in einem Lösungsmittel, wie Dichlormethan, mit Phosgen in Toluol und anschliessend mit einem Alkohol der Formel HO-($C_{2-6}$-Alkylen)-N($Y^3$,$Y^4$), worin $Y^3$ und $Y^4$ die obige Bedeutung haben, z.B. mit 1-(2-Hydroxyäthyl)-piperidin, zur entsprechenden Verbindung der Formel VII umsetzen, worin der Rest $X^3T$ in der Gruppe W eine OCOO Gruppierung ist.

Die Verbindungen der Formel II, worin einer der Reste $R^4$, $R^5$ und $R^6$ eine Aminogruppe $X^1$, $X^2$ oder $X^3$ enthält, kann man auch ausgehend von einer Verbindung der Formel

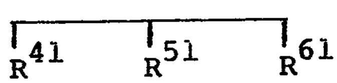

XII

herstellen, worin einer der Reste $R^{41}$, $R^{51}$ und $R^{61}$ eine Gruppe N(H,Q), ein weiterer eine Hydroxygruppe und der verbleibende z.B. eine Gruppe O-($C_{2-6}$-Alkylen)-G' ist, worin Q die gleiche Bedeutung wie $Q^1$, $Q^2$ oder $Q^3$ hat, und G' eine geschützte Hydroxygruppe, wie Trityloxy oder Benzyloxy, ist. Die Herstellung der Verbindungen XII und ihre Ueberführung in II kann man in an sich bekannter Weise durchführen, z.B. wie in den Beispielen 5A. und 7A. beschrieben.

Die Verbindungen der Formeln II, III und VIII sind neu. Als solche sind die Verbindungen der Formeln III und VIII Bestandteil der Erfindung.

Die Verbindungen der Formel I und ihre Hydrate hemmen den Blutplättchenaktivierungsfaktor (PAF) und können daher bei der Bekämpfung bzw. Verhütung von Krankheiten, wie Thrombose, Apoplexie, Herzinfarkt, Angina pectoris, Bluthochdruck und durch Allergie verursachtes Bronchialasthma, und ferner als Entzündungshemmer und Antirheumatika verwendet werden. Ferner haben die Verbindungen der Formel I und ihre Hydrate eine hemmende Wirkung auf das Wachstum von Tumorzellen und können daher als Antitumor-Mittel Verwendung finden.

Die Hemmwirkung auf PAF kann wie folgt nachgewiesen werden:

Plättchenreiches Plasma (PRP) wird durch Zentrigufation von 1/10 Volumen 90 mM Trinatriumcitrat enthaltendem Kaninchen-Blut hergestellt. Die Aggregation der Blutplättchen wird bei 37°C unter Rühren mit Hilfe eines Aggregometers gemessen. 2 Minuten nach Zugabe der Testsubstanz zum PRP wird die Plättchenaggregation durch eine submaximale Dosis von PAF (4 nM) ausgelöst. Der in der nachstehenden Tabelle angegebene $IC_{50}$-Wert (µM) entspricht derjenigen Konzentration der Testsubstanz, die die durch PAF ausgelöste Aggregation der Blutplättchen auf die Hälfte herabsetzt.

| Produkt von Beispiel: | 1 | 2 | 3 | 4 | 5 | 6 | 7 | 8 | 9 |
|---|---|---|---|---|---|---|---|---|---|
| $IC_{50}$ (µM) | 5,5 | 1,8 | 4 | 1,8 | 0,04 | 0,5 | 0,05 | 0,4 | 0,06 |

Wie eingangs erwähnt, sind Arzneimittel, enthaltend eine Verbindung der Formel I oder ein Hydrat davon, ebenfalls Gegenstand der vorliegenden Erfindung, weiterhin auch ein Verfahren zur Herstellung derartiger Arzneimittel, welches dadurch gekennzeichnet ist, dass man eine oder mehrere Ver-

bindungen der Formel I oder ein Hydrat davon und erwünschtenfalls einen oder mehrere andere therapeutisch wertvolle Stoffe in eine galenische Darreichungsform bringt.

Die Arzneimittel können enteral, z.B. oral in Form von Tabletten, Lacktabletten, Dragées, Hart- und Weichgelatinekapseln, Lösungen, Emulsionen oder Suspensionen, oder rektal, z.B. in Form von Suppositorien, oder als Spray verabreicht werden. Die Verabreichung kann aber auch parenteral, z.B. in Form von Injektionslösungen, erfolgen.

Zur Herstellung von Tabletten, Lacktabletten, Dragées und Hartgelatinekapseln kann der Wirkstoff mit pharmazeutisch inerten, anorganischen oder organischen Excipientien vermischt werden. Als solche Excipientien kann man für Tabletten, Dragées und Hartgelatinekapseln z.B. Lactose, Maisstärke oder Derivate davon, Talk, Stearinsäure oder deren Salze, verwenden. Für Weichgelatinekapseln eignen sich als Excipientien z.B. vegetabile Oele, Wachse, Fette, halbfeste und flüssige Polyole; je nach Beschaffenheit des Wirkstoffes sind jedoch bei Weichgelatinekapseln überhaupt keine Excipientien erforderlich. Zur Herstellung von Lösungen und Sirupen eignen sich als Excipientien z.B. Wasser, Polyole, Saccharose, Invertzucker und Glukose, für Injektionslösungen eignen sich z.B. Wasser, Alkohole, Polyole, Glycerin und vegetabile Oele, und für Suppositorien eignen sich z.B. natürliche oder gehärtete Oele, Wachse, Fette und halbflüssige oder flüssige Polyole.

Die pharmazeutischen Präparate können daneben noch Konservierungsmittel, Lösungsvermittler, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel oder Antioxidantien enthalten.

Die Dosierung des Wirkstoffes kann innerhalb weiter Grenzen variieren und ist natürlich in jedem einzelnen Fall den individuellen Gegebenheiten anzupassen. Im allgemeinen dürfte bei oraler Verabreichung eine Dosis von etwa 0,1 bis 20 mg/kg, vorzugsweise von etwa 0,5 bis 4 mg/kg, pro Tag für den Erwachsenen angemessen sein, wobei aber die soeben angegebene obere Grenze auch überschritten werden kann, wenn sich dies als angezeigt erweisen sollte.

## Beispiel 1

### A. Herstellung des Ausgangsmaterials

a) 15,35 g (RS)-1-O-Benzyl-3-O-tritylglycerin (36,15 mM) (Helv. Chim. Acta 65, 1982, 1059) wurden in 75 ml Chloroform gelöst. 10 ml Pyridin wurden zugefügt, gefolgt von 10,5 g Tosylchlorid. Nach 24 Stunden bei Raumtemperatur wurde das Chloroform abdestilliert. Der Rückstand wurde in 50 ml Pyridin aufgenommen. 10 ml $H_2O$ und dann 10 g $KHCO_3$ wurden zugegeben. Nach Abdestillieren der Lösungsmittel wurde der Rückstand in Toluol aufgenommen, der feste Teil wurde abgetrennt, dann wurde die organische Phase mit $H_2O$ ausgeschüttelt, getrocknet und eingedampft. Das Produkt kristallisiert aus der Schmelze beim Abkühlen, Ausbeute 95%, Smp. 98-100°C.

b) Das erhaltene (RS)-1-O-Benzyl-2-O-tosyl-3-O-tritylglycerin wurde unter Erhitzen mittels Natriumazid in trockenem DMF in das (RS)-1-O-Benzyl-2-deoxy-2-azido-3-O-tritylglycerin übergeführt.

c) Eine Lösung des erhaltenen Azids in trockenem Diäthyläther wurde mittels einer Suspension von $LiAlH_4$ in trockenem Diäthyläther zum (RS)-1-O-Benzyl-2-deoxy-2-amino-3-O--tritylglycerin, Smp. 67-69°C, reduziert.

d) Eine Lösung des erhaltenen Amins in Dioxan wurde mit verdünnter Salzsäure in das (RS)-1-O-Benzyl-2-deoxy-2-aminoglycerin-hydrochlorid, Smp. 148-149°C, übergeführt.

e) Eine wässrige Lösung des erhaltenen Hydrochlorids wird mit einer NaOH-Lösung alkalisch gestellt, das freigesetzte Amin wird in Methylenchlorid aufgenommen und das Lösungsmittel wird entfernt.

f) Einer Lösung von 1,6 g des erhaltenen (RS)-1-O-Benzyl-2-deoxy-2-aminoglycerins (8,8 mM) in 10 ml Dichlormethan wurden 2 g $K_2CO_3$ in 3 ml $H_2O$ zugefügt. Dann wurden unter Rühren 2 ml Acetanhydrid zugetropft. Nach 1 Stunde wurde das Reaktionsgemisch aufgearbeitet. Zur Reinigung wurde die Verbindung über Kieselgel mit Aether/Methanol (9:1) chromatographiert. Es wurden 1,4 g (RS)-1-O-Benzyl-2-deoxy-2-acetamidoglycerin als Flüssigkeit erhalten (81% der Theorie).

g) Einer Lösung von 1,4 g (RS)-1-O-Benzyl-2-deoxy-2-acetamidoglycerin in 10 ml Chloroform und 1,5 ml Pyridin wurden 2,1 g Stearoylchlorid in 5 ml Chloroform zugetropft. Nach 1 Stunde bei Raumtemperatur wurde das Gemisch aufgearbeitet. Zur Reinigung wurde die Verbindung über Kieselgel mit Dichlormethan/Aether (1:1) chromatographiert. Nach Kristallisation aus n-Hexan wurden 2,4 g weisse Kristalle erhalten (78% der Theorie), Smp. 65-66°C.

h) Eine Lösung von 1,5 g des erhaltenen (RS)-1-O-Stearoyl-2-deoxy-2-acetamido-3-O-benzylglycerins in 20 ml THF wurde in Anwesenheit von 0,5 g 10%iger Pd-Kohle unter leichtem $H_2$-Ueberdruck hydriert. Das (RS)-1-O-Stearoyl-2-deoxy-2-acetamidoglycerin wurde in quantitativer Ausbeute erhalten, Smp. 89-90°C (aus n-Hexan).

i) Zu einer Lösung von 1,1 g (RS)-1-O-Stearoyl-2-deoxy-2-acetamidoglycerin (2,75 mM) in 20 ml Chloroform und 0,5 ml Triäthylamin wurden im Eisbad 0,4 g 4-Chlorbuttersäurechlorid (2,8 mM) in 5 ml Chloroform unter Feuchtigkeitsausschluss getropft. Das Reaktionsgemisch wurde 2 Stunden gerührt. Nach Aufarbeitung wurde das (RS)-1-O-Stearoyl-2-deoxy-2-acetamido-3-O -(4-chlorbutyryl)glycerin aus n-Hexan kristallisiert. Man erhielt 1,39 g weisse Kristalle (Ausbeute: quantitativ). Smp. 51-52°C.

B. Herstellung des Produktes

0,45 g (RS)-1-O-Stearoyl-2-deoxy-2-acetamido-3-O -(4--chlorbutyryl)glycerin wurden mit 2 ml einer 20%igen Lösung von Trimethylamin in Acetonitril versetzt und im Bombenrohr bei 80°C während 24 Stunden zur Reaktion gebracht. Zur Reinigung wurde die Verbindung an Kieselgel mit Chloroform/-Methanol (7:3) chromatographiert. Man erhielt 0,22 g [3-[[(RS)-2-Acetamido-3-[octadecanoyloxy] propoxy]carbonyl]-propyl]trimethylammoniumchlorid (Ausbeute: 44% der Theorie), Smp. 205°C.

Beispiel 2

A. Herstellung des Ausgangsmaterials

a) Eine Lösung von 450 mg (RS)-1-O-Benzyl-2-deoxy-2-amino-glycerin-hydrochlorid in Methanol wurde mit einer Lösung von KOH in Wasser versetzt. Das Methanol wurde abdestilliert, dem Rückstand wurden Wasser und Dichlormethan zugegeben und das Gemisch wurde mit Methylchloroformiat versetzt. Man erhielt 470 mg (95%) (RS)-1-O-Benzyl-2-deoxy-2-(1-methoxyformamido)glycerin als Oel.

b) 0,45 g (RS)-1-O-Benzyl-2-deoxy-2-(1-methoxyformamido)-glycerin (1,88 mM) wurden mit 0,6 g Octadecylisocyanat versetzt und die Lösung wurde 1 Stunde auf 90°C erwärmt. Es wurde an Kieselgel mit einem Gemisch von Toluol und Essigester (4:1) chromatographiert. Nach Kristallisation aus n-Hexan erhielt man 0,65 g (RS)-1-O-Benzyl-2-deoxy-2-(1--methoxyformamido) -3-O-(octadecylcarbamoyl)glycerin, Smp. 65-67°C.

c) 4,9 g (RS)-1-O-Benzyl-2-deoxy-2-(1-methoxyformamido) -3-O-(octadecylcarbamoyl)glycerin gelöst in 75 ml THF wurden in Anwesenheit von 1 g 10%-iger Pd-Kohle, bei leichtem $H_2$-Ueberdruck hydriert. Es wurden 4,05 g (RS)-2-Deoxy-2--(1-methoxyformamido) -1-O-octadecylcarbamoylglycerin erhalten, Smp. 86°C (aus n-Hexan).

d) Zu einer Lösung von 2 g (RS)-2-Deoxy-2-(1-methoxyformamido) -1-O-octadecylcarbamoylglycerin (4,5 mM) in 20 ml Chloroform und 0,7 ml Triäthylamin wurden im Eisbad 0,75 ml 4-Chlorbuttersäurechlorid (6,68 mM) in 5 ml Chloroform unter Feuchtigkeitsausschluss getropft. Das Reaktionsgemisch wurde 2 Stunden bei Raumtemperatur gerührt. Nach Aufarbeitung wurde das Reaktionsprodukt an Kieselgel mit Dichlormethan/-Aether (1:1) filtriert. Es wurden 2,3 g (RS)-1-O-(4-Chlorbutyryl)-2-deoxy-2-(1-methoxyformamido) -3-O-(octadecylcarbamoyl)glycerin nach Kristallisation aus n-Hexan erhalten, Smp. 68-70°C.

B. Herstellung des Produktes

Analog Beispiel 1B wurden aus 0,4 g des unter A.d) erhaltenen Chlorids 0,31 g (70%) [3-[[(RS)-2-(1-Methoxyformamido) -3-(octadecylcarbamoyloxy)propoxy]carbonyl] propyl]-trimethylammoniumchlorid, Smp. 200°C (Zers.) hergestellt.

## Beispiel 3

A. Herstellung des Ausgangsmaterials

Eine Lösung von 500 mg (1,4 mMol) (RS)-2-O-Methyl-1-O--octadecylglycerin in 5 ml Dichlormethan wurde bei 0°C mit 2 ml (4 mMol) einer 20%-igen Lösung von Phosgen in Toluol versetzt. Dann wurde 3 Stunden ohne Kühlung gerührt. Der Ueberschuss an Phosgen und Dichlormethan wurde abgezogen. Dann wurde eine Lösung von 500 mg (3,9 mMol) 1-(2-Hydroxyäthyl)-piperidin in 10 ml Dichlormethan umgesetzt. Nach 2 Stunden Rühren wurde das Lösungsmittel abgedampft. Der Rückstand wurde an Kieselgel chromatographiert. Durch Eluieren mit Toluol-Aethylacetat (1:1) und dann mit Aethylacetat erhielt man das (RS)-2-O-Methyl-1-O-octadecyl -3-O-[[2--(1-piperidino)äthoxy]carbonyl]glycerin, Smp. 34°C.

B. Herstellung des Produktes

Eine Lösung von 100 mg (0,195 mMol) des unter 3A. erhaltenen Produktes in 5 ml Dichlormethan wurde mit 100 mg (0,7 mMol) Methyljodid versetzt und 3 Tage bei Raumtemperatur stehen gelassen. Das Lösungsmittel wurde abgedampft und der Rückstand mit Methanol-Dichlormethan (4:1) über chlorid-beladenem Anionenaustauscherharz perkoliert. Das Lösungsmittel wurde abgedampft und der Rückstand wurde an Kieselgel chromatographiert. Eluieren mit Chloroform-Methanol-Wasser (60:35:5) ergab 1-[2-[[[(RS)-2-Methoxy -3-(octadecyloxy)propoxy]carbonyl]oxy]äthyl] -1-methylpiperidiniumchlorid, Smp. 59°C.

Beispiel 4

A. Herstellung der Ausgangsverbindung

a) Einer Lösung von 0,9 g (RS)-1-Deoxy-1-amino-3-O-benzylglycerin (5 mM) in 20 ml Dichlormethan wurde eine wässrige KOH-Lösung zugefügt. Dem Gemisch wurde unter Rühren eine Lösung von 1,7 g Octadecylchloroformiat in 10 ml Dichlormethan zugetropft. Nach 1 Stunde Rühren bei Raumtemperatur wurde aufgearbeitet und die erhaltene Verbindung wurde an Kieselgel mit n-Hexan/Aether (1:1) chromatographiert. Nach Kristallisation aus n-Hexan erhielt man 1,6 g Kristalle, Smp. 58-69°C.

b) 2 g (RS)-1-O-Benzyl-3-deoxy -3-[1-(octadecyloxy)formamido]glycerin wurden mit 2 ml Methylisocyanat und 0,5 ml Triäthylamin während 2 Stunden auf 40°C erwärmt. Nach Entfernen des Reagenzüberschusses wurde das Produkt aus n-Hexan umkristallisiert und dabei 2 g (89,3%) (RS)-1-O-Benzyl-3-deoxy -3-[1-(octadecyloxy)formamido] -2-O-methylcarbamoylglycerin erhalten, Smp. 95-96,5°C.

c) Analog Beispiel 1A.h) und i) wurde über das (RS)-3-Deoxy-3-[1-(octadecyloxy)formamido] -2-O-methylcarbamoylglycerin das (RS)-1-O-(4-Chlorbutyryl) -3-deoxy-3-[1-(octadecyloxy)formamido] -2-O-methylcarbamoylglycerin hergestellt.

B. Herstellung des Produktes

Analog Beispiel 1B. wurde das [3-[[(RS)-2-Methylcarbamoyloxy-3-[1-(octadecyloxy)formamido]propoxy]carbonyl]propyl]trimethylammoniumchlorid hergestellt, Smp.> 200°C.

Beispiel 5

A. Herstellung der Ausgangsverbindung

a) Zu 30 ml 1,4-Butandiol und 0,2 ml $BF_3$. $Et_2O$ tropfte man unter Eisbadkühlung 5 ml Epichlorhydrin. Man liess über Nacht bei Raumtemperatur reagieren. Der Ueberschuss an Butandiol wurde zuerst abdestilliert, dann 10 g (85%) RS-1-Chlor-1-deoxy-3-O-(4-hydroxybutyl)glycerin als farblose Flüssigkeit, Sp: 110°C/0,1 mm Hg; IR ($cm^{-1}$): 3360 (OH); 1124 (Aether); 1058 (Alkohol-II Bande).

b) Das Diol von a) wurde mit Tritylchlorid in Pyridin umgesetzt. Man erhielt das RS-1-Chlor-1-deoxy-3-O-[4-(trityloxy)butyl]glycerin als viskose Flüssigkeit, IR ($cm^{-1}$): 3554 (OH); 1596 und 1490 (Aromat); 1120 (Aether); 1072 und 1032 (Alkohol); 764, 746, 706 (monosubst. Benzol).

c) Zur Herstellung von 1,2-Epoxi-3-[4-(trityloxy)butoxy]propan wurden 4,85 g des Produktes von b) in 20 ml THF gelöst und mit 2 g Kalium-t-butylat unter Rückfluss umgesetzt. Man erhielt das Produkt in quantitativer Ausbeute, IR ($cm^{-1}$): 1596 und 1490 (Aromat); 1090 und 1073 (Aether); 764, 747 und 706 (monosubst. Benzol).

d) Die Umsetzung des Epoxids von c) mit Isopropylamin im Druckkolben bei 70°C während 8 Std. ergab (RS)-3-Deoxy-3-(isopropylamino)-1-O-[4-(trityloxy)butyl]glycerin

in quantitativer Ausbeute, IR ($cm^{-1}$): 3380 und 3301 (OH und NH); 1597 und 1490 (Aromat); 1123 und 1074 (Aether); 764, 746 und 706 (monosubst. Benzol).

e) 2,4 g des Amins von d) und 2 g Octadecylchloroformiat in 20 ml $CH_2Cl_2$ wurden bei Raumtemperatur während 1. Std. in Anwesenheit von 2 ml 30%iger Kalilauge gerührt. Das Produkt wurde an Kieselgel mit Aether-Chloroform-Pyridin (49,5:49,5:1) chromatographiert. Man erhielt 3,1 g (78%) farblose Flüssigkeit, IR ($cm^{-1}$): 3421 (OH); 1696, 1669 und 1489 (Aromat); 1206 (Ester); 1124 und 1074 (Aether); 774, 764, 745 und 705 (monosubst. Benzol).

f) Die Umsetzung des Produktes von e) mit Methylisocyanat ergab (RS)-1-Deoxy-1-[N-isopropyl-1-(octadecyloxy) formamido]-2-O-(methylcarbamoyl)-3-O-[4-(trityloxy)butyl] glycerin als farblose Flüssigkeit.

g) Das Produkt von f) wurde mit wässriger HCl in Dioxan bei 95°C umgesetzt. Chromatographie an Kieselgel mit EtOAc ergab (RS)-1-Deoxy-1-[N-isopropyl-1-(octadecyloxy)formamido] -3-O-(4-hydroxybutyl)-2-O(methylcarbamoyl)glycerin als farblose Flüssigkeit, IR ($cm^{-1}$): 3353 und 3058 (NH und OH); 1702 (Carbamat); 1539 (Amid); 1255 (Ester), 1120, 1095 und 1071 (Alkohol und Aether).

h) Das Produkt von g) wurde mit Triphenylphosphindibromid in Anwesenheit von $Et_3N$ (stöchiometrische Mengen) bromiert. Aus 1,2 g Ausgangsverbindung erhielt man nach Chromatographie an Kieselgel mit Toluol-EtOAc (1:1) 0,9 g (67%) Bromid als farblose Flüssigkeit, IR ($cm^{-1}$): 3356 (NH); 1702 (Carbamat); 1530 (Amid); 1251 (NH-CO); 1132 (Aether).

B. Herstellung des Produktes

0,3 g (RS)-1-O-(4-Brombutyl)-3-deoxy-3-[N-isopropyl-3-(octacecyloxy)formamido]-2-O-(methylcarbamoyl)glycerin wurden mit 0,3 ml Thiazol versetzt und das Reaktionsgemisch wurde während 5 Std. bei 80°C gehalten. Nach Abdestillieren des überschüssigen Reagenz wurde die Verbindung an Kieselgel mit $CHCl_3$-MeOH (7:3) gereinigt. Man erhielt 0,17 g (50%) 3-[4-[(RS)-3-[N-Isopropyl-1-(octadecyloxy)formamido]-2-[(methylcarbamoyl)oxy]propoxy]butyl]thiazoliumbromid, MS: m/e = 626 ($M^+$ des Kations).

Beispiel 6

0,4 g des gleichen Ausgangsmaterials wie im Beispiel 5 wurden in 3 ml THF gelöst und mit 2 ml einer 20%igen $(CH_3)_3N$ Lösung in THF versetzt. Man liess 2 Std. im Druckkolben bei 80°C reagieren. Zur Reinigung wurde über Kieselgel mit $CHCl_3$-MeOH (1:1) filtriert. Man erhielt 0,42 g (96%) [4-[(RS)-3-[N-Isopropyl(1-octadecyloxy)formamido]-2-[(methylcarbamoyl)oxy]propoxy]butyl]trimethylammoniumbromid, MS: m/e = 600 ($M^+$ des Kations).

Beispiel 7

A. Herstellung der Ausgangsverbindung

a) Analog Beispiel 5 A. a) erhielt man aus Epichlorhydrin und 4-Benzyloxy-1-butanol das (RS)-1-O-[4-(Benzyloxy)butyl]-3-chlor-3-deoxyglycerin als farblose Flüssigkeit.

b) 15 g des Produktes von a) wurden in 20 ml Isopropylamin aufgenommen und 6 Std. im Druckkolben bei 80°C behandelt. Dann wurde der Ueberschuss Isopropylamin abdestilliert. Das gebildete Hydrochlorid wurde mit Aether gefällt und verworfen. Man erhielt das (RS)-1-O-[4-(Benzyloxy)butyl]-3-deoxy-3-(isopropylamino)glycerin, IR ($cm^{-1}$): 3302 (OH und NH); 1496 (Aromat); 1103 (Aether und Alkohol-II Bande); 737 und 698 (monosubst. Benzol).

c) Eine organische Phase bestehend aus 20 ml $CH_2Cl_2$, 5,9 g des Produktes von b) und 6,65 g Octadecylcarbamidsäurechlorid wurde 2 Std. mit 5 ml 30%iger Kalilauge gerührt. Nach Chromatographie an Kieselgel mit $CHCl_3$-EtOAc-Toluol (1:1:1) erhielt man 7,9 g (67%) (RS)-3-O-[4-(Benzyloxy)butyl]-1-deoxy-1-(1-isopropyl-3-octadecylureido)glycerin, MS (m/e): 72 (100%; = $\overset{+}{N}HCH(CH_3)_2$; 296 (2%; $O=\overset{+}{C}NH-C_{18}H_{37}$).

d) Zu einer Lösung von 3 g des Produktes von c) in 5 ml $CH_2Cl_2$ und 0,5 ml $Et_3N$ gab man 3 ml Methylisocyanat Man liess bei 40°C während 5 Std. reagieren. Das Produkt wurde an Kieselgel mit Toluol-EtOAc (1:1) gereinigt. Man erhielt 2,5 g (76%) einer wachsartigen Verbindung, IR ($cm^{-1}$): 3340 (NH); 1707 (Carbamat); 1634 (Harnstoff); 1541 (Amid-II Bande); 1101 (Aether); 735, 697 (monosubst. Benzol).

e) Die katalytische Hydrierung über 10%iger Pd-C in THF des Produkts von d) ergab (RS)-1-Deoxy-3-O-(4-hydroxybutyl)-1-(1-isopropyl-3-octadecylureido)-2-O-(methylcarbamoyl)glycerin in quantitativer Ausbeute MS (m/e): 483 (100%; $M-CH_3NHCOO$); 558 (25%; $M+H^+$).

f) Die Bromierung des Produktes von e) wurde analog Beispiel 5 A. h) durchgeführt. IR ($cm^{-1}$): 3335 (NH); 2916 und 2849 (aliph. CH); 1694 (Carbamat); 1623 (Harnstoff); 1556 und 1525 (Amid-II Bande und CO-NH offen).

B. Herstellung des Produktes

Analog Beispiel 5 B. erhielt man aus 0,3 g (RS)-3-O-(4-Brombutyl)-1-deoxy-1-(1-isopropyl-3-octadecylureido)-2-O-(methylcarbamoyl)glycerin und Thiazol 0,17 g (50%) 3-[4-[(RS)-3-(1-Isopropyl-3-octadecylureido)-2-[(methylcarbamoyl)

oxy]propoxy]butyl]thiazoliumbromid, MS (m/e): 625 ($M^+$ des Kations).

## Beispiel 8

0,2 g des gleichen Ausgangsmaterials wie in Beispiel 7 wurden in 0,5 ml N-Methylimidazol aufgenommen. Man liess bei 80°C während 30 Min. reagieren. Nach Abdestillieren des überschüssigen Reagenz wurde die Verbindung aus Methanol-Aether kristallisiert. Man erhielt 0,2 g (88%) 1-[4-[(RS)-3-(1-Isopropyl-3-octadecylureido)-2-[(methylcarbamoyl)oxy] propoxy]butyl]-3-methylimidazoliumbromid, MS (m/e): 622 ($M^+$ des Kations).

## Beispiel 9

### A. Herstellung der Ausgangsverbindung

a) Ein Gemisch von 6 g (20 mM) (RS)-1-O-(4-Benzyloxybutyl)-3-deoxy-3-isopropylaminoglycerin und 6,66 g Octadecylchloroformiat in 50 ml $CH_2Cl_2$ wurde 2 Stunden mit 20 ml 30%iger Kalilauge gerührt. Nach Isolierung des Produktes aus der organischen Phase und Chromatographie an Kieselgel mit Toluol-Essigester (4:1) erhielt man 11 g (91,5%) (RS)-1-O-(4-Benzyloxybutyl)-3-deoxy-3-(N-isopropyl-1-octadecyloxyformamido)glycerin, IR ($cm^{-1}$): 3431 (OH), 1696 und 1670 (Carbamat), 1119 (Aether und Alkohol-II Bande); 734 und 697 (monosubst. Benzol).

b) 4,5 g des Produktes aus a) wurden mit Methylchloroformiat in Anwesenheit von Pyridin zu 3,8 g (80%) (RS)-1-O-(4-Benzyloxybutyl)-3-deoxy-2-O-methoxycarbonyl-3-(N-isopropyl-1-octadecyloxyformamido)glycerin verestert, IR ($cm^{-1}$): 1751 (Carbonat C=O), 1701 (Carbamat C=O), 1266 (Ester), 1101 (Aether); 734 und 697 (monosubst. Benzol).

c) 3,65 g des Produktes aus b) wurden über 10%iger Pd-C in THF zu 3,1 g (94%) (RS)-1-Deoxy-3-O-(4-hydroxybutyl)-1-(N-isopropyl-1-octadecyloxyformamido)-2-O-methoxycarbonyl-glycerin hydriert, Smp. 28°C.

d) Analog Beispiel 5A.h wurden 2,9 g des Produktes von c) zu 2,5 g (77,5%) (RS)-1-O-(4-Brombutyl)-3-deoxy-3-(N-iso-propyl-1-octadecyloxyformamido)-2-O-methoxycarbonylglycerin bromiert, IR ($cm^{-1}$): 1751 (Carbonat CO),1700 (Carbamat CO), 1266 (Ester), OH-Bande verschwunden.

B. Herstellung des Produktes

Analog Beispiel 5B wurden 1,5 g des Produktes von A.d mit 1 ml Thiazol zu 1,45 g (85%) 3-[4-[(RS)-3-(N-Isopropyl-1-octadecyloxyformamido)-2-(methoxycarbonyloxy)propoxy]-butyl]thiazoliumbromid umgesetzt, Smp. 124-125°C (aus einem Gemisch von 2 ml Aceton und 30 ml Aether).

## Beispiel A

Eine Verbindung der Formel I kann man in an sich bekannter Weise als Wirkstoff zur Herstellung von Tabletten folgender Zusammensetzung verwenden:

| | pro Tablette |
|---|---|
| Wirkstoff | 200 mg |
| mikrokristalline Cellulose | 155 mg |
| Maisstärke | 25 mg |
| Talk | 25 mg |
| Hydroxypropylmethylcellulose | 20 mg |
| | 425 mg |

## Beispiel B

Eine Verbindung der Formel I kann man in an sich bekannter Weise als Wirkstoff zur Herstellung von Kapseln folgende Zusammensetzung verwenden:

| | pro Kapsel |
|---|---|
| Wirkstoff | 100,0 mg |
| Maisstärke | 20,0 mg |
| Milchzucker | 95,0 mg |
| Talk | 4,5 mg |
| Magnesiumstearat | 0,5 mg |
| | 220,0 mg |

## Patentansprüche

1. Glycerinderivate der Formel

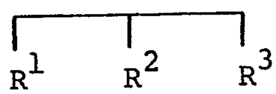 I

worin einer der Reste $R^1$, $R^2$ und $R^3$ eine Gruppe U der Formel $OY^1$ oder $-X^1-CO-(A^1)_n-Z^1$,
ein weiterer eine Gruppe V der Formel
$OY^2$ oder $-X^2-CO-(A^2)_p-Z^2$,
und der verbleibende eine Gruppe W der Formel
$-X^3T-(C_{2-6}\text{-Alkylen})-N^+R\ .\ A^-$ ist,
wobei eines von $X^1$, $X^2$ und $X^3$ Sauerstoff oder eine Gruppe $NQ^1$ ist und die zwei anderen Sauerstoff sind,

$Y^1$ $C_{10-26}$-Alkyl oder $C_{10-26}$-Alkenyl,

$Y^2$ $C_{1-6}$-Alkyl, $C_{2-6}$-Alkenyl, $C_{3-6}$-Cycloalkyl, $C_{5-6}$-Cycloalkenyl, Phenyl, Benzyl oder 2-Tetrahydropyranyl,

$A^1$ und $A^2$ Sauerstoff oder eine Gruppe $NQ^2$,

n und p die Zahl 1 oder 0,

$Z^1$ $C_{9-25}$-Alkyl oder $C_{9-25}$-Alkenyl,

$Z^2$ $C_{1-5}$-Alkyl, $C_{2-5}$-Alkenyl, Phenyl oder, falls $A^2$ nicht Sauerstoff ist, auch Wasserstoff bedeutet,

T Carbonyl, COO oder $CONQ^3$ oder, falls $X^3$ Sauerstoff ist, auch Methylen bedeutet,

$Q^1$, $Q^2$ und $Q^3$ Wasserstoff, $C_{1-4}$-Alkyl, $C_{3-6}$-Cycloalkyl oder Phenyl,

$A^-$ das Anion einer starken Säure,

$-N^+R$ eine Gruppe $-N^+(Y^3,Y^4,Y^5)$ oder, falls zumindest eines von $X^1$, $X^2$, $X^3$, $A^1$, $A^2$ und T ein substituiertes N-Atom enthält, auch eine am quartären Stickstoff gebundene 5- oder 6-gliedrige aromatische Gruppe, gegebenenfalls mit einem zusätzlichen Heteroatom O, S oder N, gegebenenfalls mit ankondensiertem Benzol und gegebenenfalls monosubstituiert durch Alkyl oder Alkoxy mit bis zu 4 C-Atomen, Hydroxy, Nitro, Carbamoyl oder Ureido, $Y^3$ und $Y^4$ $C_{1-6}$-Alkyl oder zusammen $C_{3-6}$-Alkylen, und $Y^5$ $C_{1-6}$-Alkyl sind,

wobei, falls gleichzeitig $R^1$ eine Gruppe $OY^1$ oder $OY^2$, und $R^3$ eine Gruppe W ist, worin $X^3$ Sauerstoff und T Methylen oder Carbonyl bedeutet, $R^2$ die Gruppe OCOO oder ein substituiertes N-Atom enthält,

und deren Hydrate.

2. Verbindungen nach Anspruch 1, worin $R^1$ eine Gruppe U, $R^2$ eine Gruppe V und $R^3$ eine Gruppe W ist.

3. Verbindungen nach Anspruch 1 oder 2, worin $R^1$ Octadecanoyloxy, Octadecylcarbamoyloxy, Octadecyloxy, Octadecyloxyformamido, N-Isopropyloctadecyloxyformamido oder 1-Isopropyl-3-octadecylureido ist.

4. Verbindungen nach Anspruch 1, 2 oder 3, worin $R^2$ Acetamido, Methoxyformamido, Methoxy, Methylcarbamoyloxy oder Methoxycarbonyloxy ist.

5. Verbindungen nach einem der Ansprüche 1-4, worin $R^3$ [4-(Trimethylammonio)-n-butyryloxy]chlorid, [2-(1-Methylpiperidinio)äthoxycarbonyloxy]chlorid, [4-(3-Thiazolio)-n-butoxy]bromid, [4-(Trimethylammonio)-n-butoxy]bromid oder [4-(3-Methylimidazolio)-n-butoxy]bromid ist.

6\. Verbindungen nach einem der Ansprüche 1-5, worin $R^1$ Octadecanoyloxy, Octadecylcarbamoyloxy, Octadecyloxy, Octadecyloxyformamido, N-Isopropyloctadecyloxyformamido oder 1-Isopropyl-3-octadecylureido, $R^2$ Acetamido, Methoxyformamido, Methoxy, Methylcarbamoyloxy oder Methoxycarbonyloxy, $R^3$ [4-(Trimethylammonio)-n-butyryloxy]chlorid, [2-(1-Methylpiperidinio)äthoxycarbonyloxy]chlorid, [4-(3-Thiazolio)-n-butoxy]bromid, [4-(Trimethylammonio)-n-butoxy]bromid oder [4-(3-Methylimidazolio)-n-butoxy]bromid ist.

7\. Verbindungen nach einem der Ansprüche 1-6 zur Verwendung als therapeutischen Wirkstoff, insbesondere als Hemmer des Blutplättchenaktivierungsfaktors oder des Wachstums von Tumoren.

8\. Verfahren zur Herstellung der Verbindungen nach Anspruch 1, dadurch gekennzeichnet, dass man

a) eine Verbindung der Formel

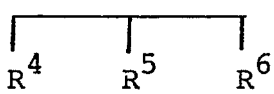

II

worin die Reste $R^4$, $R^5$ und $R^6$ die gleiche Bedeutung wie die Reste $R^1$, $R^2$ bzw. $R^3$ haben, wobei jedoch in der Gruppe W an Stelle des Restes $-N^+R$ $A^-$ eine Abgangsgruppe vorliegt,

mit einem Amin der Formel NR umsetzt, oder

b) eine Verbindung der Formel

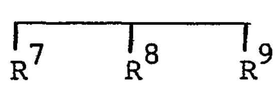

III

worin die Reste $R^7$, $R^8$ und $R^9$ die gleiche Bedeutung wie die Reste $R^1$, $R^2$ bzw. $R^3$ haben, wobei jedoch an Stelle einer der Gruppen U und V eine Hydroxy- oder eine Aminogruppe vorliegt,

mit einer Säure der Formel

$Z^1-(A^1)_n-COOH$ oder $Z^2-(A^2)_p-COOH$ IV

oder einem reaktiven Derivat davon, oder mit einem Isocyanat der Formel

$Z^1NCO$ oder $Z^2NCO$ V

oder einem Imidazolid der Formel

$Z^1N(H)C(O)$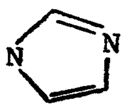 oder $Z^2N(H)C(O)$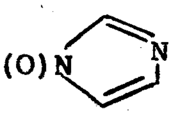 IV

umsetzt, oder

c) eine Verbindung der Formel

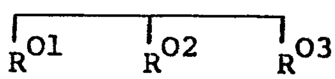 VII

worin $R^{O1}$, $R^{O2}$ und $R^{O3}$ die gleiche Bedeutung wie die Reste $R^1$, $R^2$ bzw. $R^3$ haben, wobei jedoch in der Gruppe W an Stelle der Gruppe $-N^+R$ $A^-$ eine Gruppe $-N(Y^3,Y^4)$ vorliegt,

mit einem $C_{1-6}$-Alkylhalogenid umsetzt, wobei $R^1$, $R^2$, $R^3$, $-N^+R$, $A^-$, U, V, W, $Z^1$, $Z^2$, $A^1$, $A^2$, $Y^3$, $Y^4$, n und p die oben angegebene Bedeutung haben.

9. **Pharmazeutisches Präparat auf der Basis einer Verbindung nach einem der Ansprüche 1-6.**

10. **Verwendung der Verbindungen nach einem der Ansprüche 1-6 zur Herstellung eines Mittels zur Hemmung des Blutplättchenaktivierungsfaktors** oder des Wachstums von Tumoren.

***

Patentansprüche für Oesterreich

1. Verfahren zur Herstellung von Glycerinderivaten der Formel

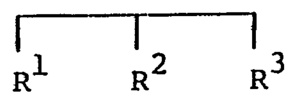

I

worin einer der Reste $R^1$, $R^2$ und $R^3$ eine Gruppe U der Formel $OY^1$ oder $-X^1-CO-(A^1)_n-Z^1$, ein weiterer eine Gruppe V der Formel $OY^2$ oder $-X^2-CO-(A^2)_p-Z^2$, und der verbleibende eine Gruppe W der Formel $-X^3T-(C_{2-6}\text{-Alkylen})-N^+R\ \ A^-$ ist, wobei eines von $X^1$, $X^2$ und $X^3$ Sauerstoff oder eine Gruppe $NQ^1$ ist und die zwei anderen Sauerstoff sind,

$Y^1$ $C_{10-26}$-Alkyl oder $C_{10-26}$-Alkenyl,

$Y^2$ $C_{1-6}$-Alkyl, $C_{2-6}$-Alkenyl, $C_{3-6}$-Cycloalkyl, $C_{5-6}$-Cycloalkenyl, Phenyl, Benzyl oder 2-Tetrahydropyranyl,

$A^1$ und $A^2$ Sauerstoff oder eine Gruppe $NQ^2$,

n und p die Zahl 1 oder 0,

$Z^1$ $C_{9-25}$-Alkyl oder $C_{9-25}$-Alkenyl,

$Z^2$ $C_{1-5}$-Alkyl, $C_{2-5}$-Alkenyl, Phenyl oder, falls $A^2$ nicht Sauerstoff ist, auch Wasserstoff bedeutet,

T Carbonyl, COO oder $CONQ^3$ oder, falls $X^3$ Sauerstoff ist, auch Methylen bedeutet,

$Q^1$, $Q^2$ und $Q^3$ Wasserstoff, $C_{1-4}$-Alkyl, $C_{3-6}$-Cycloalkyl oder Phenyl,

$A^-$ das Anion einer starken Säure,

$-N^+R$ eine Gruppe $-N^+(Y^3,Y^4,Y^5)$ oder, falls zumindest eines von $X^1$, $X^2$, $X^3$, $A^1$, $A^2$ und T ein substituiertes N-Atom enthält, auch eine am quartären Stickstoff gebundene 5- oder 6-gliedrige aromatische Gruppe, gegebenenfalls mit einem zusätzlichen Heteroatom O, S oder N, gegebenenfalls mit ankondensiertem Benzol und gegebenenfalls monosubstituiert durch Alkyl oder Alkoxy mit bis zu 4 C-Atomen, Hydroxy, Nitro, Carbamoyl oder Ureido,
$Y^3$ und $Y^4$ $C_{1-6}$-Alkyl oder zusammen $C_{3-6}$-Alkylen,
und $Y^5$ $C_{1-6}$-Alkyl sind,
wobei, falls gleichzeitig $R^1$ eine Gruppe $OY^1$ oder $OY^2$, und $R^3$ eine Gruppe W ist, worin $X^3$ Sauerstoff und T Methylen oder Carbonyl bedeutet, $R^2$ die Gruppe OCOO oder ein substituiertes N-Atom enthält,
und deren Hydrate, dadurch gekennzeichnet, dass man

a) eine Verbindung der Formel

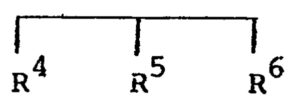 II

worin die Reste $R^4$, $R^5$ und $R^6$ die gleiche Bedeutung wie die Reste $R^1$, $R^2$ bzw. $R^3$ haben, wobei jedoch in der Gruppe W an Stelle des Restes $-N^+R$ $A^-$ eine Abgangsgruppe vorliegt,
mit einem Amin der Formel NR umsetzt, oder

b) eine Verbindung der Formel

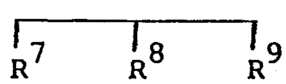 III

worin die Reste $R^7$, $R^8$ und $R^9$ die gleiche Bedeutung wie die Reste $R^1$, $R^2$ bzw. $R^3$ haben, wobei jedoch an Stelle einer der Gruppen U und V eine Hydroxy- oder eine Aminogruppe vorliegt,

mit einer Säure der Formel

$Z^1-(A^1)_n-COOH$ oder $Z^2-(A^2)_p-COOH$ IV

oder einem reaktiven Derivat davon, oder mit einem Isocyanat der Formel

$Z^1NCO$ oder $Z^2NCO$ V

oder einem Imidazolid der Formel

$Z^1N(H)C(O)$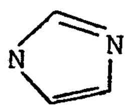 oder $Z^2N(H)C(O)$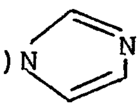 IV

umsetzt, oder

c) eine Verbindung der Formel

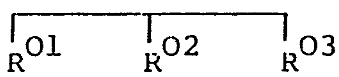 VII

worin $R^{O1}$, $R^{O2}$ und $R^{O3}$ die gleiche Bedeutung wie die Reste $R^1$, $R^2$ bzw. $R^3$ haben, wobei jedoch in der Gruppe W an Stelle der Gruppe $-N^+R$ $A^-$ eine Gruppe $-N(Y^3,Y^4)$ vorliegt,
mit einem $C_{1-6}$-Alkylhalogenid umsetzt, wobei $R^1$, $R^2$, $R^3$, $-N^+R$, $A^-$, U, V, W, $Z^1$, $Z^2$, $A^1$, $A^2$, $Y^3$, $Y^4$, n und p die oben angegebene Bedeutung haben.

2. Verfahren nach Anspruch 1, worin $R^1$ eine Gruppe U, $R^2$ eine Gruppe V und $R^3$ eine Gruppe W ist.

3. Verfahren nach Anspruch 1 oder 2, worin $R^1$ Octadecanoyloxy, Octadecylcarbamoyloxy, Octadecyloxy, Octadecyloxyformamido, N-Isopropyloctadecyloxyformamido oder 1-Isopropyl-3-octadecylureido ist.

4. Verfahren nach Anspruch 1, 2 oder 3, worin $R^2$ Acetamido, Methoxyformamido, Methoxy, Methylcarbamoyloxy oder Methoxycarbonyloxy ist.

5. Verfahren nach einem der Ansprüche 1-4, worin $R^3$ [4-(Trimethylammonio)-n-butyryloxy]chlorid, [2-(1-Methylpiperidinio)äthoxycarbonyloxy]chlorid, [4-(3-Thiazolio)-n-butoxy]bromid, [4-(Trimethylammonio)-n-butoxy]bromid oder [4-(3-Methylimidazolio)-n-butoxy]bromid ist.

6. Verfahren nach einem der Ansprüche 1-5, worin $R^1$ Octadecanoyloxy, Octadecylcarbamoyloxy, Octadecyloxy, Octadecyloxyformamido, N-Isopropyloctadecyloxyformamido oder 1-Isopropyl-3-octadecylureido, $R^2$ Acetamido, Methoxyformamido, Methoxy, Methylcarbamoyloxy oder Methoxycarbonyloxy, $R^3$ [4-(Trimethylammonio)-n-butyryloxy]chlorid, [2-(1-Methylpiperidinio)äthoxycarbonyloxy]chlorid, [4-(3-Thiazolio)-n-butoxy]bromid, [4-(Trimethylammonio)-n-butoxy]bromid oder [4-(3-Methylimidazolio)-n-butoxy]bromid ist.